# EUROPEAN PATENT SPECIFICATION

(11) **EP 3 704 134 B1**
(45) Date of publication and mention of the grant of the patent: **22.12.2021**
(21) Application number: 18795520.8
(22) Date of filing: 29.10.2018
(51) Int. Cl.: C07K 5/117, A61K 38/07, A61K 8/64, A61K 38/00, A61Q 15/00

(54) **ANTIMICROBIAL TETRAPEPTIDES**
ANTIMIKROBISCHE TETRAPEPTIDE
TETRAPEPTIDES ANTIMICROBIENS

(30) Priority: 30.10.2017 EP 17199080
(43) Date of publication of application: 09.09.2020
(73) Proprietor: DSM IP Assets B.V., 6411 TE Heerlen (NL)
(72) Inventor: HEIDL, Marc, 4303 Kaiseraugst (CH); JACKSON, Eileen, 4303 Kaiseraugst (CH)
(74) Representative: Berg, Katja
(86) International application number: PCT/EP2018/079522
(87) International publication number: WO 2019/086361

(56) References cited:
- WO-A2-2014/081845
- KOIKOV L N ET AL: "Sub-Nanomolar hMC1R Agonists by End-Capping of the Melanocortin Tetrapeptide His-D-Phe-Arg-Trp-NH2", BIOORGANIC & MEDICINAL CHEMISTRY LETTERS, PERGAMON, AMSTERDAM, NL, vol. 13, 1 January 2003 (2003-01-01), pages 2647-2650, XP002342336, ISSN: 0960-894X, DOI: 10.1016/S0960-894X(03)00552-3
- HOLDER J R ET AL: "Characterization of aliphatic, cyclic, and aromatic N-terminally "capped" His-D-Phe-Arg-Trp-NH2 tetrapeptides at the melanocortin receptors", EUROPEAN JOURNAL OF PHARMACOLOGY, ELSEVIER SCIENCE, NL, vol. 462, 21 February 2003 (2003-02-21), pages 41-52, XP002263329, ISSN: 0014-2999, DOI: 10.1016/S0014-2999(03)01322-0
- LAU QIU YING ET AL: "Discovery of an ultra-short linear antibacterial tetrapeptide with anti-MRSA activity from a structure-activity relationship study", EUROPEAN JOURNAL OF MEDICINAL CHEMISTRY, EDITIONS SCIENTIFIQUE ELSEVIER, PARIS, FR, vol. 105, 22 October 2015 (2015-10-22), pages 138-144, XP029290685, ISSN: 0223-5234, DOI: 10.1016/J.EJMECH.2015.10.015

## Description

The present invention relates to novel tetrapeptides as well as the use thereof as antimicrobial agents.

Antimicrobial active compounds play a key role for many cosmetic applications:
Acne is taken to mean a skin disorder which is evident in inflamed papules, pustules or nodules, caused by increased talc production and impaired keratinization of the
skin. The inflammation may be associated with reddening, swelling and pressure pain. Besides genetic predisposition, possible causes of acne formation can be androgens, comedogenic substances (for example in cosmetics), smoking, stress or excessive colonization of the skin by bacteria. Acne is in particular triggered by the microorganism Propionibacterium acnes (P. acnes), which is a bacterium which usually colonizes the skin and lives on sebum. Acne may arise, for example, if the number of these bacteria is increased. The presence of bacteria in the follicles results in inflammation reactions, which is evident in the form of red nodules or pustules. The production of free fatty acids by the bacteria furthermore promotes the inflammation reaction in the follicle. While many approaches to acne treatment have been reported, some have advocated use of a systemic or topical agent to address the overgrowth of Propionibacterium acnes.

The fundamental basis of axillary or foot odor is the sweat of the skin. Sweat itself is odorless. However, sweat together with other components from the skin as are some fatty acids and elements from the desquamated cells are used as nutrients for microorganisms which are part of the normal skin flora. By metabolizing these compounds from the skin, volatile organic compounds are released malodorous. Bacillus subtilis, for example, was shown to be closely associated with increased foot odor.

Skin infections are the most common effects of a staphylococcus aureus (S. aureus) overpopulation. These infections can start as a simple crusting of the skin, known as impetigo, which often starts with redness of the skin and then progresses into bullae, which is an elevation of the skin filled with fluid. Other skin infections caused by S. aureus are folliculitis, an inflammation of a hair follicle; furuncle, a small abscess affecting the skin and subcutaneous tissues; and carbuncle, a collection of furuncles

Topical antibiotics which have been utilized to attempt to inhibit the overgrowth of microorganisms on the skin are clindamycin, erythromycin, tetracycline, and metronidazole. Each of these topical antibiotics reportedly cause side effects and widespread use also contributes to the risk of bacterial resistance.

Thus, there is an ongoing need for non-antibiotic alternatives, which can help to (selectively) control the growth of (certain) microorganism on the skin and are thus suitable for the treatment of any ailments associated with their overpopulation.

Surprisingly it has been found that compounds of formula (I) 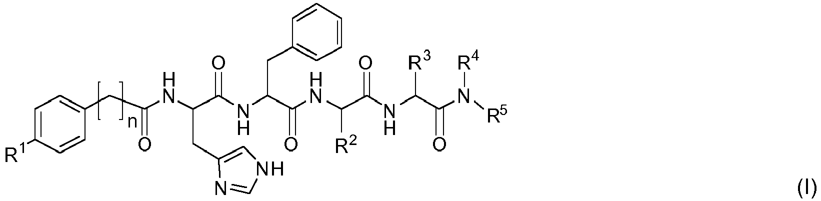 wherein
R¹ is selected from a C₁-C₆alkyl group or a C₁-C₆alkoxy group,
R² is an amino acid side chain of a basic amino acid,
R³ is an arylC₁-C₆alkyl group or a heteroarylC₁-C₆alkyl group,
R⁴ and R⁵ are, independently of each other H, an arylC₁-C₆alkyl group or a C₁-C₁₀alkyl group, wherein the alkyl group is optionally substituted with up to three hydroxy groups, and n is an integer selected from 0 to 3
or a cosmetically acceptable salt thereof are suitable antimicrobial agents to inhibit the growth of B. subtilis, and optionally P. acnes and/ or S. aureus and are thus particularly suitable for the incorporation into cosmetic compositions to treat any ailments resulting from an overpopulation thereof on the skin.

Thus, in a first aspect, the present invention relates to a compound of formula (I) 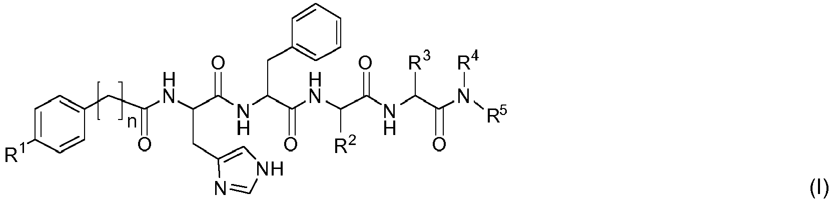 wherein
R¹ is selected from H, a C₁-C₆ alkyl group or a C₁-C₆alkoxy group,
R² is an amino acid side chain of a basic amino acid,
R³ is an arylC₁-C₆alkyl group or a heteroarylC₁-C₆alkyl group,
R⁴ and R⁵ are, independently of each other H, an arylC₁-C₆alkyl group or a C₁-C₁₀alkyl group, wherein the alkyl group is optionally substituted with up to three hydroxy groups, and n is an integer selected from 0 to 3,
or a cosmetically acceptable salt thereof.

In all embodiments of the present invention, advantageously the following compounds of formula (I) are excluded (disclaimed):
- PhCO-His-(D-Phe)-Arg-Trp-NH₂
- PhCH₂CO-His-(D-Phe)-Arg-Trp-NH₂
- Ph(CH₂)₂CO-His-(D-Phe)-Arg-Trp-NH₂
- Ph(CH₂)₃CO-His-(D-Phe)-Arg-Trp-NH₂
- 4-ButoxyPhCO-His-(D-Phe)-Arg-Trp-NH₂
- Ph(CH₂)₃CO-His-(D-Phe)-Arg-Trp-NHEt
- Ph(CH₂)₃CO-His-(D-Phe)-Arg-Trp-NHMe

Even more preferably the present invention relates to compounds of formula (I) with the proviso that if R² is the amino acid side chain of arginine and R³ is (1H-indol-3-yl)methyl then
(1) if R⁴ and R⁵ are H and n is an integer from 0 to 3, then R¹ is not H, or
(2) if R⁴ and R⁵ are H and n is 0 then R¹ is not butoxy, or
(3) if R¹ and R⁴ are H and n is 3, then R⁵ is not methyl or ethyl.

The term 'C₁-C₆alkyl group', respectively 'C₁-C₁₀alkyl group' refers to unbranched C₁-C₆alkyl respectively C₁-C₁₀alkyl or branched C₃-C₆alkyl respectively C₃-C₁₀alkyl groups which may be optionally substituted by up to three hydroxy groups such as methyl, ethyl, n-propyl, 1-methylethyl, 3-hydroxypropyl, 2,3-dihydroxypropyl, 3-hydroxybutyl, 4-hydroxybutyl n-butyl, 1-methylpropyl, 2-methylpropyl, 1,1-dimethylethyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl and 3,5,5-trimethylhexyl groups, 2,3-dihydroxypropyl such as preferably methyl, ethyl, propyl, butyl, hexyl, heptyl, octyl or 2,3-hydroxypropyl.

The term 'C₁-C₆alkoxy group' refers to unbranched C₁-C₆alkoxy groups or to branched C₃-C₆alkoxy groups such as methoxy, ethoxy, propoxy, isopropoxy, butyloxy or tert-butyloxy groups.

The term "aryl" as used herein refers to an aromatic substituent containing 5 to 15 carbon atoms and containing a single aromatic ring or multiple aromatic rings which are fused together, directly linked or indirectly linked (such that the different aromatic rings are bound to a common group such as a methylene or ethylene group). Particularly advantageous aryl groups according to the present invention contain 5 to 12 carbon atoms containing a single aromatic ring or multiple aromatic rings which are fused together. Most preferred aryl residues in all embodiments of the present invention are phenyl, naphthyl and biphenyl.

The term "side chain" of an amino acid refers to that portion of the amino acid attached to the common NH₂-CH-COOH backbone of the respective amino acids. For instance, the side chain of serine is -CH₂-OH and the side chain of alanine is -CH₃.

The term "basic amino acid" as used herein refers to any natural or unnatural amino acid that have basic side chains at neutral pH such as the natural occurring amino acids arginine (Arg), lysine (Lys), and histidine (His) as well as the unnatural amino acids 2,4-diaminobutyric acid, homolysine and ornithine without being limited thereto. Advantageous amino acid side in all embodiments of the present invention are the side chains of arginine, lysine, 2,4-diaminobutyric acid, homolysine and ornithine such as in particular the side chains of arginine and 2,4-diaminobutyaric acid.

The term "arylC₁-C₆alkyl group" as used herein refers to a -C₁-C₆ alkyl-aryl group (i.e. to a C₁-C₆alkyl group which is substituted by an aryl group, i.e. the attachment point is via the alkyl group), wherein the term "aryl" is as defined above. Advantageous arylC₁-C₆alkyl groups are arylC₁-C₂alkyl such as in particular phenyl(m)ethyl or naphthyl(m)ethyl.

The term 'heteroarylC₁-C₆alkyl group' refers to a -C₁-C₆alkyl-heteroaryl group (i.e. to a C₁-C₆alkyl group which is substituted by a heteroaryl group, i.e. the attachment point is via the alkyl group) wherein the term "heteroaryl" refers to a 5- or 6-membered aromatic ring containing one or more heteroatoms, viz., N, O or S; these heteroaromatic rings may be fused to other aromatic systems. Particularly preferred heteroaromatic rings encompass indole, pyridine and quinoline. In all embodiments of the present invention preferred heteroarylC₁-C₆alkyl group are heteroarylC₁-C₂alkyl groups such as (1H-indol-3-yl)(m)ethyl, (pyridin-2-yl)(m)ethyl, (pyridin-3-yl)(m)ethyl, (quinolin-2-yl)(m)ethyl and (quinolin-3-yl)(m)ethyl groups.

The aromatic aryl respectively heteroaryl residue in the heteroarylC₁-C₆alkyl groups may be unsubstituted or substituted with one or more substituents. In all embodiments of the present invention, such substituents are preferably selected from halogen, hydroxy, nitro, cyano, C₁-C₆alkyl, C₁-C₆alkoxy and C₁-C₆alkanoyloxy. More preferably in all embodiments of the present invention the heteroaryl residues are unsubstituted or substituted with one substituent selected from the group consisting of F, Cl, hydroxy, cyano, C₁-C₃alkyl, C₁-C₃alkoxy and C₁-C₃alkanoyloxy. Most preferably, in all embodiments of the present invention, the heteroaryl residues are unsubstituted.

In all embodiments of the present invention particular preferred heteroarylC₁-C₂alkyl groups are (1H-indol-3-yl)(m)ethyl, 5-fluoro(1H-indol-3-yl)(m)ethyl, 6-fluoro(1H-indol-3-yl)(m)ethyl, 5-hydroxy(1H-indol-3-yl)(m)ethyl, (pyridin-2-yl)(m)ethyl, (pyridin-3-yl)(m)ethyl, (quinolin-2-yl)(m)ethyl and (quinolin-3-yl)(m)ethyl. Most preferred in all embodiments of the present invention are the heteroarylC₁-alkyl groups (1H-indol-3-yl)methyl, 5-fluoro(1H-indol-3-yl)methyl, 6-fluoro(1H-indol-3-yl)8m)ethyl, 5-hydroxy(1H-indol-3-yl)methyl, (pyridin-2-yl)methyl, (pyridin-3-yl)methyl, (quinolin-2-yl)methyl and (quinolin-3-yl)methyl, such as most preferably(1H-indol-3-yl)methyl.

It is well understood, that the present invention encompasses the compounds of formula (I) as optically pure isomers such as e.g. as pure enantiomers or stereoisomers as well as mixtures of different isomers such as e.g. as racemates, or mixtures of diastereoisomers.

Particularly preferred in all embodiments of the present invention, however, are compounds of formula (I) which are compounds of formula (I-A) with the stereochemistry as depicted below

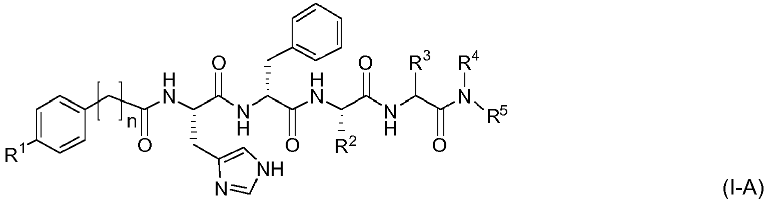

Most preferred in all embodiments of the present invention are compounds of formula (I) or (I-A) wherein R¹ is selected from H or a C₁-C₂alkyl group or a C₁-C₂alkoxy group, even more preferably R¹ is H or (m)ethoxy, such as in particular H or methoxy.

Most preferred in all embodiments of the present invention are compounds of formula (I) or (I-A) wherein R² is the amino acid side chain of arginine or diaminobutyric acid.

Most preferred in all embodiments of the present invention are compounds of formula (I) or (I-A) wherein R³ is an aryl(m)ethyl group or an heteroaryl(m)ethyl group, even more preferably phenyl(m)ethyl, naphthyl(m)ethyl or (1H-indol-3-yl)(m)ethyl such as in particular phenylmethyl, naphthylmethyl or (1H-indol-3-yl)methyl.

Most preferred in all embodiments of the present invention are compounds of formula (I) or (I-A) wherein R⁴ and R⁵ are, independently of each other H, an arylC₁-C₆alkyl group or a C₁-C₁₀alkyl group, wherein the alkyl group may be substituted with up to two hydroxyl groups, such as even more preferably H, benzyl or an unbranched C₁-C₁₀alkyl group, wherein the alkyl group may be substituted with up to two hydroxyl groups, such as in particular H, benzyl, propyl, butyl, octyl or 2,3-hydroxypropyl.

Most preferred in all embodiments of the present invention are compounds of formula (I) or (I-A) wherein n is preferably 2 or 3, even more preferably n is 3.

The term 'or a cosmetically acceptable salt thereof' refers to compounds of formula (I) or (I-A) in the form of an acid addition salt such as in the form of a chloride, an acetate or a trifluoroacetate salt. Alternatively, the salt may be formed by reaction with an alkali or earth alkaline base resulting in the respective alkali or earth alkaline salt such as in particular the respective lithium, sodium, potassium, magnesium or calcium salts.

Most preferred, in all embodiments of the present invention, are the compounds of formula (I) or (I-A) with all the definitions and preferences as given herein as such or in the form of their acetates or trifluoroacetates (i.e. as 2,2,2-trifluoroacetates). Such salts are easily prepared by a person skilled in the art.

In a particular advantageous embodiment, the present invention relates to compounds of formula (I-A), which are compounds of formula (II), 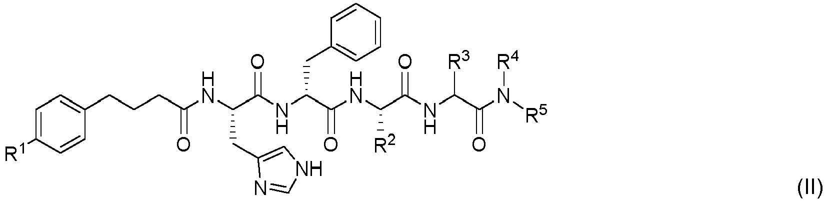 wherein
R¹ is selected from H, C₁-C₂alkyl group or a C₁-C₂alkoxy group, such as preferably H or a C₁-C₂alkoxy group,
R² is an amino acid side chain of a basic amino acid, such as preferably the amino acid side chain of arginine or diaminobutyric acid,
R³ is an aryl(m)ethyl group or an heteroaryl(m)ethyl group, such as preferably phenyl(m)ethyl, naphthyl(m)ethyl or (1H-indol-3-yl)(m)ethyl,
R⁴ and R⁵ are, independently of each other H, benzyl or a C₁-C₁₀alkyl group, wherein the alkyl group may be substituted with up to two hydroxyl groups, such as preferably H, benzyl or an unbranched C₁-C₁₀alkyl group, wherein the alkyl group may be substituted with up to two hydroxyl groups, or
a cosmetically acceptable salt thereof, such as preferably an acetate or a trifluoroacetate.

Even more advantageous compounds of formula (I-A) or cosmetically acceptable salts thereof in all embodiments of the present invention are compounds of formula (III), 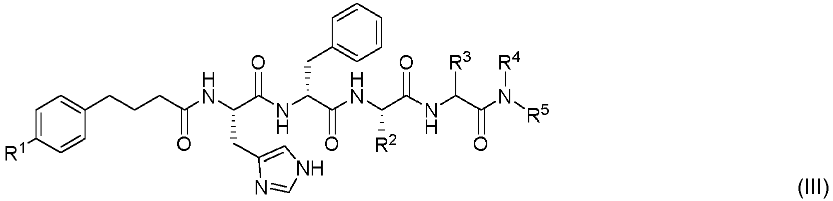 wherein
R¹ is H or methoxy,
R² is the amino acid side chain of arginine or diaminobutyric acid,
R³ is naphthylmethyl or (1H-indol-3-yl)methyl, and
R⁴ and R⁵ are, independently of each other selected from the group consisting of H, benzyl, propyl, butyl, octyl or 2,3-hydroxypropyl.

It is well understood, that the above-mentioned disclaimer and proviso for the compounds of formula (I) also apply to the compounds of formula (I-A), (II) and (III), in so far as the compounds are still encompassed within the respective Markush structure.

Most preferred in all embodiments according to the present invention are the compounds of formula (I-A), which are compounds of formula (I-a) to (I-h) and which are listed in table 1

**Table 1:**

| **#** | **Structure** | **Notation based on amino acid sequence*** |
|---|---|---|
| | 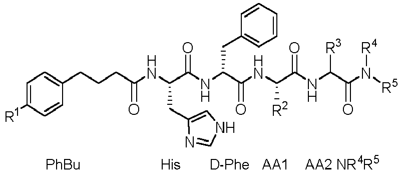 | PhBu-His-D-Phe-AA1-AA2-NR⁴R⁵ |
| (I-a) | 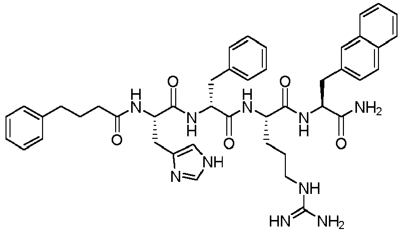 | PhBu-His-D-Phe-Arg-L-2NaphAla-NH₂ *2TFA |
| (I-b) | 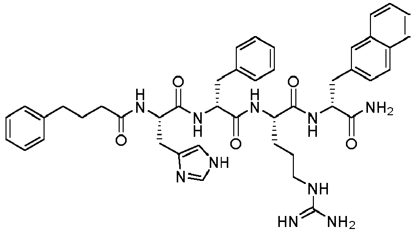 | PhBu-His-D-Phe-Arg-D-2NaphAla-NH₂ *2TFA |
| (I-c) | 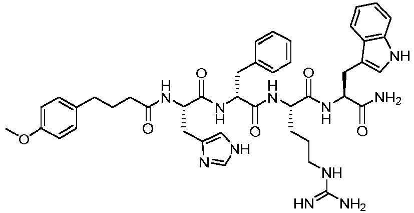 | 4-MeO-PhBu-His-D-Phe-Arg-Trp-NH₂ × 2 AcOH |
| (I-d) | 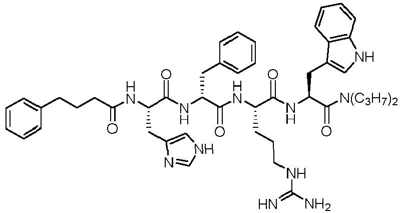 | PhBu-His-D-Phe-Arg-Trp-N(Propyl)₂ *2TFA |
| (I-e) | 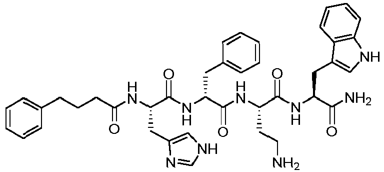 | PhBu-His-D-Phe-Dab-Trp-NH₂ *2TFA |
| (I-f) | 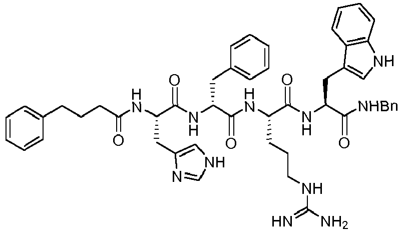 | PhBu-His-D-Phe-Arg-Trp-NH-Bn*2TFA |
| (I-g) | 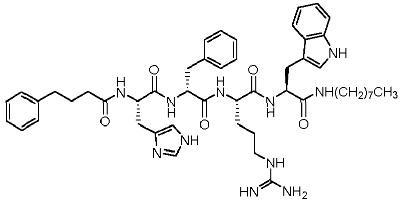 | PhBu-His-D-Phe-Arg-Trp-NH-Octyl * 2 TFA |
| (I-h) | 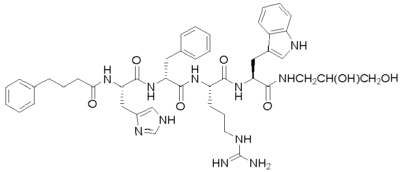 | PhBu-His-D-Phe-Arg-Trp-NH-CH₂-CH(OH)-CH₂OH *2TFA |

The compounds according to the present invention may be prepared from by methods standard in peptide chemistry as illustrated in the examples.

In yet another embodiment the present invention relates to the use of a compound of formula (I), (I-A), (II), (III) and (I-a) to (I-h) according to the present invention and with all the definitions and preferences as given herein as antimicrobial agent, such as in particular as antimicrobial agent against B. subtilis, and optionally in addition against P. acnes and/ or S. aureus.

The present invention also relates to a method for method for killing and/ or inhibiting growth of microbial cells, in particular bacterial cells such as most preferably B. subtilis and optionally P. acnes and/ or S. aureus, said method comprising contacting said microbial cells with a compound of formula (I), (I-A), (II), (III) and (I-a) to (I-h) according to the present invention.

The term "antimicrobial activity" (or "antimicrobial effect") as used herein means a capability of killing and/or inhibiting the growth of microbial cells such as in particular bacteria more in particular B. subtilis and optionally in addition P. acnes and/ or S. aureus.

Due to the antimicrobial activity, the compounds of formula (I), (I-A), (II), (III) and (I-a) to (I-h) according to the present invention are particularly suitable to maintain a healthy skin homeostasis and/ or balance the skin microbiome by treating overpopulation of microorganisms on the skin such as B. subtilis (antiperspirant/ deodorant applications) and/or optionally in addition P. acnes (acne control application) and by reducing unwanted microorganisms such as S. aureus.

The present invention furthermore relates to the use of a compound of formula (I), (I-A), (II), (III) and (I-a) to (I-h) according to the present invention as deodorant and/ or as anti-acne compound.

Also advantageous is the use of the compounds of formula (I), (I-A), (II), (III) and (I-a) to (I-h) according to the present invention as active compound in deodorants or antiperspirants as all of them excerpt an antimicrobial action against B. subtilis which is responsible for the decomposition of sweat and thus for the formation of the unpleasant odor. In a particular advantageous embodiment the compounds of formula (I), (II), (III) and (I-a) to (I-h) according to the present invention are used to treat foot malodor.

Furthermore, the compounds of formula (I), (I-A), (II), (III) and (I-a) to (I-h) according to the present invention may also be suitable for the treatment or prophylaxis of acne which is triggered by P. acnes.

The use according to the invention of the compounds of formula (I), (I-A), (II), (III) and (I-a) to (I-h) according to the present invention can take place both in the cosmetic sense and in the pharmaceutical sense. A pharmaceutical application is conceivable, for example, in the case of anti-acne compositions. In all embodiments of the present invention, the use is however preferably cosmetic (non-therapeutic).

Thus, in another embodiment, the invention relates to a cosmetic or pharmaceutical composition comprising at least one compound of formula (I), (I-A), (II), (III) and (I-a) to (I-h) according to the present invention and a cosmetically acceptable carrier.

The cosmetic or pharmaceutical compositions according to the present invention are in particular topically applied to mammalian keratinous tissue such as in particular to human skin or the human scalp and hair.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

The amount of the compound of formula (I) in the cosmetic or pharmaceutical composition can be easily adjusted by a person skilled in the art in order to achieve the desired beneficial effect. Preferably, the amount of the compound of formula (I), (II), (III) and (I-a) to (I-h) according to the present invention in the cosmetic or pharmaceutical compositions according to the present invention is at least 1 ppm based on the total weight of the cosmetic composition. In all embodiments of the present invention the amount of the compound of formula (I) is preferably selected in the range of about 0.00001 to 0.5 wt.-%, more preferably in the range of 0.0001 to 0.25 wt.-%, most preferably in the range of 0.0001 to 0.1 wt.-% based on the total weight of the cosmetic composition.

In another embodiment, the present invention also relates to a method to reduce malodor on skin, said method comprising the step of topically applying a cosmetic composition according to the present invention with all the definitions and preferences given herein to the skin.

The amount of the cosmetic composition to be applied to the skin is not critical and can easily be adjusted by a person skilled in the art. Preferably the amount is selected in the range of 0.1 to 3 mg/cm² skin, such as preferably in the range of 0.1 to 2 mg/cm² skin and most preferably in the range of 0.5 to 2 mg/cm² skin.

The term 'cosmetic composition' refers to compositions which are used to treat, care for or improve the appearance of the skin and/or the scalp. Particular advantageous cosmetic compositions according to the present invention are skin care compositions.

The cosmetic or pharmaceutical compositions according to the invention are preferably intended for topical application, which is to be understood as the external application to keratinous substances, such as in particular the skin.

The term 'cosmetically acceptable carrier' as used herein refers to a physiologically acceptable medium which is compatible with keratinous substances. Suitable carriers are well known in the art and are selected based on the end-use application. Preferably, the carriers of the present invention are suitable for application to skin (e.g., sunscreens, creams, milks, lotions, masks, serums, hydrodispersions, foundations, creams, creamgels, or gels etc.). Such carriers are well-known to one of ordinary skill in the art, and can include one or more compatible liquid or solid filler diluent, excipient, additive or vehicle which are suitable for application to skin. The exact amount of carrier will depend upon the level of the compound of formula (I) and any other optional ingredients that one of ordinary skill in the art would classify as distinct from the carrier (e.g., other active components). The compositions of the present invention preferably comprise from about 75% to about 99.999%, more preferably from about 85% to about 99.99%, still more preferably from 90% to about 99%, and most preferably, from about 93% to about 98%, by weight of the composition, of a carrier.

The cosmetic or pharmaceutical compositions of the present invention can be formulated into a wide variety of product types, including creams, waxes, pastes, lotions, milks, mousses, gels, oils, tonics, and sprays. Preferably the compounds of formula (I) are formulated into lotions, creams, gels, and tonics. These product forms may be used for a number of applications, including, but not limited to, hand and body lotions, facial moisturizers, anti-ageing preparations, make-ups including foundations, and the like. Any additional components required to formulate such products vary with product type and can be routinely chosen by one skilled in the art.

If compositions of the present invention are formulated as an aerosol and applied to the skin as a spray-on product, a propellant is added to the composition.

The cosmetic or pharmaceutical compositions according to the present invention can be prepared by conventional methods in the art such as e.g. by admixing a compound of formula (I) with all the definitions and preferences given herein with the cosmetically acceptable carrier. The cosmetic compositions of the invention (including the carrier) may comprise further conventional cosmetic adjuvants and additives, such as preservatives/antioxidants, fatty substances/oils, water, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the cosmetic or pharmaceutical compositions according to the invention may also comprise further cosmetically active ingredients conventionally used in cosmetic compositions. Exemplary active ingredients encompass further self-tanning agents, UV-filters, agents for the treatment of hyperpigmentation; agents for the prevention or reduction of inflammation; firming, moisturizing, soothing, and/ or energizing agents as well as agents to improve elasticity and skin barrier.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the cosmetic excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The compositions according to this invention can additionally comprise further organic or inorganic UV-filter substances (light screening agents) which are active in the UV-A and/or UV-B regions (absorbers), such UV-filter substances being water- soluble, fat-soluble or insoluble in commonly used cosmetic solvents.

Exemplary UVA, UVB and/ or broadspectrum UV-filter substances encompass dibenzoylmethane derivatives such as e.g. butyl methoxydibenzoylmethane (PARSOL^{®} 1789); acrylates such as e.g. octocrylene (PARSOL^{®} 340); camphor derivatives such as e.g. 4-methyl benzylidene camphor (PARSOL^{®} 5000) or terephthalylidene dicamphor sulfonic acid (Mexoryl^{®} SX); cinnamate derivatives such as e.g. ethylhexyl methoxycinnamate (PARSOL^{®} MCX) or isoamyl methoxycinnamate; p-aminobenzoic acid derivatives such as e.g. p-aminobenzoic acid or 2-ethylhexyl p-dimethylaminobenzoate; benzophenones such as e.g. benzophenone-3, benzophenone-4, 2,2',4,4'-tetrahydroxybenzophenone or 2,2'-dihydroxy-4,4'-dimethoxybenzophenone; esters of benzalmalonic acid such as e.g. di-(2-ethylhexyl) 4-methoxybenzalmalonate; organosiloxane compounds carrying chromophore groups such as e.g. polysilicone-15 (PARSOL^{®} SLX) or drometrizole trisiloxane (Mexoryl^{®} XL); imidazole derivatives such as e.g. 2-phenyl benzimidazole sulfonic acid and salts thereof such as e.g. its sodium- or potassium salts (PARSOL^{®} HS); salicylate derivatives such as e.g. ethylhexyl salicylate (PARSOL^{®} EHS, Neo Heliopan^{®} OS), isooctyl salicylate or homosalate (PARSOL^{®} HMS, Neo Heliopan^{®} HMS); triazine derivatives such as e.g. ethylhexyl triazone (Uvinul^{®} T-150), diethylhexyl butamido triazone (Uvasorb^{®} HEB), bis-ethylhexyloxyphenol methoxyphenyl triazine (Tinosorb^{®} S) or Tris-Biphenyl Triazine (2,4,6-Tris(biphenyl-4-yl)-1,3,5-triazin, Tinosorb^{®} A2B); Benzotriazole derivatives such as e.g. methylene bis-benzotriazolyl tetramethylbutylphenol (Tinosorb^{®} M); encapsulated UV-filters such as e.g. encapsulated ethylhexyl methoxycinnamate (Eusolex^{®} UV-pearls); amino substituted hydroxybenzophenones such as e.g. diethylamino hydroxybenzoyl hexyl benzoate (Aminobenzophenon, Uvinul^{®} A Plus); benzoxazol-derivatives such as e.g. 2,4-bis-[5-1(dimethylpropyl)benzoxazol-2-yl-(4-phenyl)-imino]-6-(2-ethylhexyl)-imino-1 ,3,5-triazin (Uvasorb^{®} K2A); phenylene-1,4-bis-benzimidazolsulfonic acids or salts thereof such as e.g. disodium phenyl dibenzimidazole tetrasulfonate (2,2-(1,4-phenylene)bis-(1H-benzimidazol-4,6-disulfonic acid, Neoheliopan^{®} AP); 1,1'-(1,4-piperazinediyl)bis[1-[4-(diethylamino)-2-hydroxybenzoyl]phenyl]-methanone (CAS No. 919803-06-6); as well as Bis(butylbenzoate) diaminotriazine aminopropyltrisiloxane (CAS No. 207562-42-3).

Inorganic UV-filter substances encompass pigments such as e.g. microparticulated zinc oxide or titanium dioxide (e.g. commercially available as PARSOL^{®} TX) The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.

Preferred UVB-filter substances to be incorporated into the cosmetic compositions according to the invention encompass polysilicone-15, phenylbenzimidazol sulfonic acid, octocrylene, ethylhexyl methoxycinnamate, ethyl hexylsalicylate, tris-biphenyl triazine and/ or homosalate.

Preferred broadband UV-filter substances to be incorporated into the cosmetic compositions according to the invention encompass unsymmetrical s-triazine derivatives such as in particular bis-ethylhexyloxyphenol methoxyphenyl triazine, certain benzophenones such as e.g. 2-hydroxy-4-methoxy-benzophenon, methylene bis-benzotriazolyl tetramethylbutylphenol and/ or titanium dioxide.

Preferred UVA-filter substances to be incorporated into the cosmetic compositions according to the invention encompass butyl methoxydibenzoylmethane, diethylamino hydroxybenzoyl hexyl benzoate, 2,4-bis-[5-1(dimethylpropyl)benzoxazol-2-yl-(4-phenyl)-imino]-6-(2-ethylhexyl)-imino-1,3,5-triazine and/ or disodium phenyl dibenzimidazole tetrasulfonate, in particular butyl methoxydibenzoylmethane and/ or diethylamino hydroxybenzoyl hexyl benzoate.

If the topical sunscreen emulsions comprise butyl methoxydibenzoylmethane, then they advantageously contain in addition at least one suitable photostabilizer for butyl methoxydibenzoylmethane. Besides specific UV-filters listed above which are known to a person skilled in the art to be able to photostabilize butyl methoxydibenzoylmethane, further exemplary photostabilizers encompass Polyester 8 (Polycrylene^{®}); Methoxycrylene (Solastay); diethylhexyl syringylidene malonate (Oxynex ST liquid); diethylhexyl naphthalate (Corapan TQ) as well as Benzotriazolyl Dodecyl p-Cresol (Tinogard^{®} TL) without being limited thereto. An overview on such photostabilizers is e.g. given in 'SPF Boosters & Photostability of Ultraviolet Filters', HAPPI, October 2007, p. 77-83 which is included herein by reference. These photostabilizers are generally used in an amount of 0.05 to 10 wt.-% with respect to the total weigh of the topical sunscreen emulsion.

If present, the amount of the UV-filter substances (i.e. the sum of all UV-filter substances present in the cosmetic composition) is preferably selected in the range of 0.1 to 40 wt. %, more preferably in the range of 0.2 to 20 wt. % and most preferably in the range of 0.5 to 15 wt.-% based on the total weight of the cosmetic composition.

In a particular embodiment, the cosmetic compositions according to the present invention may further comprise at least one additional self-tanning agent which is preferably selected from the group consisting of mono- or polycarbonyl compounds, such as, for example, isatin, alloxan, ninhydrin, glyceraldehyde, mesotartaric aldehyde, glutaraldehyde, erythrulose, the pyrazoline-4,5-dione derivatives as described in FR-2,466,492 and WO97/35842, dihydroxyacetone (DHA) or the 4,4-dihydroxy-pyrazolin-5-one derivatives as described in EP-903,342. Preferably, DHA and/or erythrulose (in D- or L-form or as the racemate) in particular erythrulose.

DHA can be used in the free form and/or in the encapsulated form, for example encapsulated in lipid vesicles, such as liposomes, which are described, in particular, in WO 97/25970.

The cosmetic compositions according to the present invention may also contain at least one synthetic or natural direct dye and/or at least one indole derivative, such as those described in EP-425,324 and EP-456,545 and/ or at least one synthetic or natural agent for coloring the skin.

By the term "agent for coloring the skin" is intended any compound having a specific affinity for the skin and which imparts thereto a lasting and noncovering (namely, having no tendency to opacify the skin) coloring, which is removed neither with water nor using a solvent, and which withstands both rubbing and washing with a solution comprising surfactants. Such a lasting coloring is therefore distinguished from the superficial and short-lived coloring contributed, for example, by a makeup pigment.

The additional coloring agents can also be selected, for example, from among plant extracts, such as, for example, extracts of "insoluble" redwoods of the Pzerocarpus genus and of the Baphia genus, such as Pzerocarpus santalinus, Pterocarpus osun, Plerocarpus soyauxii, Plerocarpus erinaceus, Pterocarpus indicus or Baphia nitida, such as those described in EP-971,683.

The coloring agents can also be iron oxide nanopigments for which the mean size of the individual particles is less than 100 nm, such as those described in EP-966,953.

If present, the total amount of such additional self-tanning agent(s) in the compositions according to the invention is generally selected in proportions ranging from 0.1% to 20% by weight with respect to the total weight of the composition and preferably from 0.2% to 8% by weight with respect to the total weight of the composition.

Particularly preferred are cosmetic or pharmaceutical compositions according to the present invention which further comprise at least on ingredient selected from the group consisting of polysilicones-15, phenylbenzimidazol sulfonic acid, 3-benzylidene camphor, octocrylene, ethylhexyl methoxycinnamate, ethyl hexylsalicylate, homosalate, zinc oxide, bis-ethylhexyloxyphenol methoxyphenyl triazine, methylene bis-benzotriazolyl tetramethylbutylphenol, titanium dioxide, butyl methoxydibenzoylmethane, erythrulose, potassium cetyl phosphate, tocopherol and/ or tocopherol acetate.

The cosmetic compositions according to the present invention may be in the form of a suspension or dispersion in solvents or fatty substances, or alternatively in the form of an emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, as masks or as sprays.

If the cosmetic composition is an emulsion, such as in particular an O/W, W/O, Si/W, W/Si, O/W/O, W/O/W multiple or a pickering emulsion, then the amount of the oily phase present in such cosmetic emulsions is preferably at least 10 wt.-%, such as in the range of 10 to 60 wt.-%, preferably in the range of 15 to 50 wt.-%, most preferably in the range of 15 to 40 wt.-%, based on the total weight of the cosmetic composition.

In one embodiment, the cosmetic compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the cosmetic composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol^{®} A from DSM Nutritional Products Ltd.), diethanolamine cetyl phosphate (e.g. as Amphisol^{®} DEA from DSM Nutritional Products Ltd.), potassium cetyl phosphate (e.g. as Amphisol^{®} K from DSM Nutritional Products Ltd.), sodium cetearylsulfate, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the cosmetic composition.

Particular suitable O/W emulsifiers to be used in the cosmetic compositions according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate.

A particular suitable O/W emulsifier to be used in the cosmetic compositions according to the invention is potassium cetyl phosphate e.g. commercially available as Amphisol^{®} K at DSM Nutritional Products Ltd Kaiseraugst.

Another particular suitable class of O/W emulsifiers are non-ionic self-emulsifying systems derived from olive oil e.g. known as (INCI Name) cetearyl olivate and sorbitan olivate (chemical composition: sorbitan ester and cetearyl ester of olive oil fatty acids) sold under the tradename OLIVEM 1000.

In one particular embodiment, the invention relates to cosmetic compositions with all the definitions and preferences given herein in the form of O/W emulsions comprising an oily phase dispersed in an aqueous phase in the presence of an O/W emulsifier wherein the O/W emulsifier is potassium cetyl phosphate. The amount of oily phase in such O/W emulsions is preferably at least 10 wt.-%, more preferably in the range of 10 to 60 wt.-%, most preferably in the range of 15 to 50 wt.-%, such as in the range of 15 to 40 wt.-%.

The cosmetic compositions according to the invention in general have a pH in the range of 3 to 10, preferably a pH in the range of 4 to 8 and most preferably a pH in the range of 4 to 7.5. The pH can easily be adjusted as desired with suitable acids, such as e.g. citric acid, or bases, such as sodium hydroxide (e.g. as aqueous solution), triethanolamine (TEA Care), Tromethamine (Trizma Base) and Aminomethyl Propanol (AMP-Ultra PC 2000), according to standard methods in the art.

Further suitable uses of the compounds according to the present invention encompass pharmaceutical applications. Thus, the compounds according to the present invention may be used to prepare a pharmaceutical composition for the treatment, prevention and/or prophylaxis of any disorder and disease where it is desirable to inhibit/ kill B. subtilis, P. acnes and/ or S. aureus in a patient in need thereof. The compounds may be applied topical, oral as well as parenteral without being limited thereto.

The invention is further illustrated with reference to the following, non-limiting examples, in which all percentages are by weight based on total weight unless otherwise specified.

### Experimental part

### General Information

### Abbreviations:

- AA: Amino acid
- Arg: arginine
- Boc: tert-butyloxycarbonyl
- Dab: 2,4 diaminobutyric acid
- DCM: dichloromethane
- DIPEA: *N,N*-di*i*sopropylethylamine
- DMAP: *N,*N-dimethylaminopyridine
- DMF: dimethylformamide
- Fmoc: fluorenylmethoxycarbonyl
- Gly: Glycine
- His: Histidine
- HPLC: High Pressure Liquid Chromatography
- IPE: Di iso propyl ether
- NaphAla: Naphthylalanin
- PhBu: 4-phenyl butyric acid
- Phe: phenylalanin
- TBTU: O-(Benzotriazol-1-yl)-N,N,N',N'-tetramethyluronium tetrafluoroborat
- TCTU: 2-(2-Pyridon-1-yl)-1,1,3,3-tetramethyluronium tetrafluoroborate
- TFA: trifluoroacetic acid
- TIPS: triisopropylsilane
- Trp: Tryptophan
- Trt: Trityl

*Preparative HPLC purifications*: performed on a Waters High Performance Liquid Chromatography LC-2525 equipped with a Waters 2767 Sample Manager and a Waters FCII automated fraction collector, using a Grom Saphir 110 C18 10 µm 50×300 mm² preparative column and a Waters 2487 double wavelength UV-Vis detector operating at 220 and 254 nm.
H₂O + 0.07 % TFA (A" phase) and MeCN + 0.07 % TFA (B" phase) were used as eluents, with a flow of 55 mL/min.

### Synthetic strategies

Unsubstituted amides were prepared on rink linker amide resin using solid phase synthesis approach. After simultaneous cleavage of the side chain protecting groups as well as the attachment to the resin, the crude peptide is purified by preparative HPLC. Substituted amides were prepared on 2-chloro trityl resin side as chain protected peptides with free acid and coupled with the corresponding amine to give the side chain protected test item in solution, which is deprotected and thoroughly purified by HPLC.

### Preparation

### 1. Free amides:

Typical procedure: approx. 2.0 g Fmoc-ramage-resin (loading approx. 0.5 mmol/g) is placed in a peptide synthesizer reaction tube and the sequence is assembled on a peptide synthesizer. 1.25 eq of the respective Fmoc-amino acids (side-chain functional groups are Boc/Pbf/Trt protected if present) are coupled to the growing peptide chain with 1.25 eq TBTU and 3 eq DIPEA. Fmoc protection group is removed with 4 methyl piperidine. The phenylbutyric acid or its derivative is coupled on the peptide using the standard peptide coupling procedure.

The fully assembled peptide is cleaved from the resin with 25.8 ml of a TFA/TIPS/DCM = 22.5/0.8/2.5 mixture (v/v). The crude peptide is precipitated by adding the solution to 200ml of IPE/Hexan = 1/1 (v/v). The precipitate is directly purified by preparative HPLC. The acidic modifier of the liquid phase matches the desired salt form (acetic acid for I-c, TFA for all others)

**Table 2**

| Entry | Sequence | Amount, yield |
|---|---|---|
| I-a | PhBu-His-D-Phe-Arg-L-2NaphAla-NH2 *2TFA | 425 mg (41%) |
| I-b | PhBu-His-D-Phe-Arg-D-2NaphAla-NH2 *2TFA | 307 mg (31 %) |
| I-c | 4-MeO-PhBu-His-D-Phe-Arg-Trp-NH2 × 2 AcOH | 386 mg (13%) |
| I-e | PhBu-His-D-Phe-Dab-Trp-NH2 *2TFA | 374 mg (37%) |

### Substituted amides

Typical procedure: approx. 2 g 2-chloro-trityl-resin (loaded with the fist amino acid approx. 0.5 mmol/g) is placed in a peptide synthesizer reaction tube and the sequence is assembled on a peptide synthesizer. 1.25 eq of the respective Fmoc-amino acids (side-chain functional groups are Boc/Pbf/Trt protected if present) are coupled to the growing peptide chain with 1.25 eq TBTU and 3 eq DIPEA. Fmoc protection group is removed with 4 methyl piperidine. The phenylbutyric acid is coupled on the peptide using the standard peptide coupling procedure.

The fully assembled peptide is cleaved from the resin with three times 20 ml of DCM containing 0.1% TFA. The combined DCM portions are combined in a separatory funnel and washed neutral. Organic phase is dried over Na₂SO₄ and all volatile compounds removed in vacuum. Crude peptide is carefully coupled using 3 eq of 2,4,6-trimethyl-pyridine 1 eq of TPTU and 1.1 eq amine at 0°C. Regular aqueous workup (NaHCO₃, KHSO₄, NaCl) is followed by removal of all side chain protecting groups with TFA/TIPS/DCM = 22.5/0.8/2.5 mixture (v/v) and precipitation by adding the solution to IPE/Hexan = 1/1 (v/v). The precipitate is directly purified by preparative HPLC.

**Tanle 3**

| Entry | Sequence | Amount, yield |
|---|---|---|
| I-d | PhBu-His-D-Phe-Arg-Trp-N(Propyl)₂ *2TFA | 262 mg (33%) |
| I-f | PhBu-His-D-Phe-Arg-Trp-NH-Bn *2TFA | 209 mg (11%) |
| I-g | PhBu-His-D-Phe-Arg-Trp-NH-Octyl *2TFA | 279 mg (28%) |
| I-h | PhBu-His-D-Phe-Arg-Trp-NH-CH₂-CH(OH)-CH₂OH *2TFA | 227 mg (18%) |

### 3. Antimicrobial activity

The peptides are dissolved in culture medium at 400ppm. A control in the microbiological medium alone and a control consisting of Phenonip^{®} prepared to 5% (v/v) directly in the microbiological medium was used.

The determination of minimal inhibitory concentration of each product is performed according to a liquid media method adapted in 96-wells plates.

To determine the minimum inhibitory concentration (MIC), the peptides are tested in serial dilutions (½ to ½ for 8 dilutions) directly in the liquid culture medium to favor the growth of bacteria in microplate 96 wells. Then, each point of dilution is contaminated with each strain at about 7×10⁵ cfu/ml for S. aureus (ATCC 6538), 5,5 ×10⁵cfu/ml for P. acnes (ATCC 11827) and 1,2 ×10⁵cfu/ml for B. subtilis (ATCC 6633) per well. Finally, plates are incubated 48 hours at 32, 5 °C ± 2.5 °C, taking care to respect the respiratory type of each strain.

At the end of the incubation time of 48 hours, the presence or absence of turbidity reveals the state of microbial growth. The last dilution corresponding to the absence of bacterial growth is taken as minimum inhibitory concentration (MIC) for the microbial strain considered. The results of the study are presented in the following table:

**Table 4: MIC values**

| # | Peptide | B. subtilis | S. aureus | P. acnes |
|---|---|---|---|---|
| I-a | PhBu-His-D-Phe-Arg-L-2NaphAla-NH₂ *2TFA | 62.5 ppm | 125 ppm | 500 ppm |
| I-b | PhBu-His-D-Phe-Arg-D-2NaphAla-NH₂ *2TFA | 100 ppm | - | 400 ppm |
| I-c | 4-MeO-PhBu-His-D-Phe-Arg-Trp-NH₂ × 2 AcOH | 200 ppm | - | - |
| I-d | PhBu-His-D-Phe-Arg-Trp-N(Propyl₎₂ *2TFA | 15.6 ppm | 31.3 ppm | 125 ppm |
| I-e | PhBu-His-D-Phe-Dab-Trp-NH₂ *2TFA | 400 ppm | - | - |
| I-f | PhBu-His-D-Phe-Arg-Trp-NH-Bn*2TFA | 252 ppm | 400 ppm | - |
| I-g | PhBu-His-D-Phe-Arg-Trp-NH-Octyl *2TFA | 252 ppm | 200 ppm | - |
| I-h | PhBu-His-D-Phe-Arg-Trp-NH-CH₂-CH(OH)-CH₂OH *2TFA | 317 ppm | - | - |
| | Phenonip^{®} | 790 ppm | 630 ppm | 630 ppm |

As can be seen, all peptides exhibited a significant antimicrobial activity against B. subtilis, which is even better than the one of Phenonip^{®}, a well-known cosmetic antimicrobial agent. Some of the peptides also inhibited (selectively) the growth of P. acnes and/ or S. aureus.

### Cosmetic composition

Table 5 outlines exemplary O/W emulsions, wherein one compound selected from the group of (I-h) as outlined in table 1, is incorporated in the indicated amount.

**Table 5: Exemplary O/W emulsion**

| **O/W Emulsions** | **1** | **2** | **3** | **4** | **5** | **6** | **7** | **8** |
|---|---|---|---|---|---|---|---|---|
| Glyceryl Stearate | 2.5 | 2 | 1.2 | 1 | | | 1 | 1 |
| PEG-40 Stearate | 1 | | | | | | | |
| PEG-100 Stearate | | 2.5 | | | | | | 1 |
| Ceteareth-20 | | | | | 1 | | | |
| Glyceryl Stearate Citrate | | | | | | 0.5 | | |
| Potassium Cetyl Phosphate | | | | | | | 3 | 1.5 |
| Stearic Acid | | | 2.5 | 3 | | | | |
| Cetearyl Alcohol | 4 | | | 2 | | | 2 | |
| Stearyl Alcohol | | 2 | 1 | | | | | |
| Cetyl Alcohol | | | 1 | 1 | | | | 0.5 |
| Acrylates/C₁₀₋₃₀ Alkyl Acrylate Crosspolymer | | | | 0.2 | 0.2 | 0.4 | 0.2 | |
| Carbomer | 0.1 | | 0.2 | | | | | |
| Xanthan Gum | | 0.3 | | | | | | 0.3 |
| C₁₂₋₁₅ Alkyl Benzoate | 5 | | | 2 | 5 | 5 | 10 | 5 |
| Petrolatum | 5 | | 3 | | | | | |
| Butylene Glycol Dicaprylate/Dicaprate | | 4 | 2 | | 9 | | | 9 |
| Hydrogenated Polydecene | | | 3 | | 2 | | | 2 |
| Caprylic/Capric Triglyceride | 1 | 3 | | 5 | | 5 | 5 | |
| Cyclomethicone | | 5 | 2 | | | 10 | | |
| Methylpropanediol | 2 | | | | 3 | | | 3 |
| Glycerine | 4 | 7 | 3 | 4 | 3 | | 5 | 3 |
| Glyceryl Glucoside | 3.5 | 3 | 1 | 1 | 2 | | | 2 |
| Alcohol denat. | 1 | 3 | 0.5 | 10 | 4 | 8 | | 4 |
| Butylene Glycol | | | 3 | | | | | |
| Ascorbylglucoside | | 0.5 | | 1.0 | | 1.5 | | 0.1 |
| Ubiquinone (Coenzyme 10) | 0.1 | | 0.05 | | | | 0.01 | |
| Hyaluronic acid | | | | | 0.2 | | | |
| Bisabolol | 0.5 | | | | | | 0.2 | |
| Isotridecylsalicylate | | | 1 | 3 | 5 | 2 | 3 | 5 |
| Compound selected from the group of (I-a) to (I-h) | 0.001 | 0.25 | 0.0001 | 0.05 | 0.1 | 0.0003 | 0.03 | 0.002 |
| Dibutyl Adipate | 1.5 | 3 | | | | | | |
| Diisopropyl sebacate | | 1 | 1 | 2 | 3 | | | |
| Ethylhexyl Benzoate | | | | | | 0.75 | 1.5 | 1 |
| Titanium Dioxide (PARSOL TX) | | | 0.5 | 2 | | | | |
| Methylene Bis-Benztriazoyl Tetramethylbutylphenol | | | 0.5 | 4 | | 6 | | 2 |
| Ethylhexyl methoxycinnamate | | | | | 2 | | | |
| Phenylbenzimidazole Sulfonic Acid | | | | 2 | | 2 | 2 | |
| Butyl Methoxydibenzoylmethane | | 1 | | 2 | 2 | 3 | 3 | 3 |
| Methylbenzylidene Camphor | | | | | 2 | 3 | | |
| Octocrylene | | 5 | | | | 2 | 10 | |
| Polysilicone-15 | | | | 2 | | 3 | | |
| Ethylhexyl Salicylate | | | | | 5 | | | |
| Homosalate | | | 4 | | 2 | | | |
| Bis-Ethylhexyloxyphenol Methoxyphenyltriazine | | 1.5 | | | | | | 2 |
| Erythrulose | | | | 1 | | | 1 | |
| Dihydroxyacetophenone | 1 | | | | | | 0.5 | 0.5 |
| Silica | 1 | | 2.5 | | | 0.5 | | |
| Silica & Methicone | | 4 | | 1 | 2.5 | | | |
| Methyl Methacrylate Crosspolymer | | | | 1 | | | 2 | |
| Disodium EDTA | 0.1 | | | | | 0.5 | | |
| Fragrance, Preservatives | q.s. | | | | | | | |
| Sodium Hydroxide | q.s. | | | | | | | |
| Water | Ad 100 | | | | | | | |

## Claims

1. A compound of formula (I) 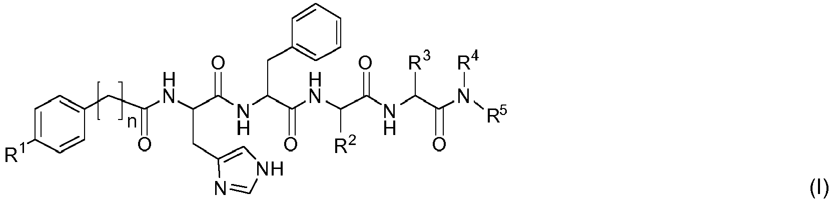 wherein
R¹ is selected from H, a C₁-C₆alkyl group or a C₁-C₆alkoxy group,
R² is an amino acid side chain of a basic amino acid,
R³ is an arylC₁-C₆alkyl group or a heteroarylC₁-C₆alkyl group,
R⁴ and R⁵ are, independently of each other H, an arylC₁-C₆alkyl group or a C₁-C₁₀alkyl group, wherein the alkyl group is optionally substituted with up to three hydroxy groups, and
n is an integer selected from 0 to 3,
with the proviso that
if R² is the amino acid side chain of arginine and R³ is (1H-indol-3-yl)methyl then
(1) if R⁴ and R⁵ are H and n is an integer from 0 to 3, then R¹ is not H, or
(2) if R⁴ and R⁵ are H and n is 0, then R¹ is not butoxy, or
(3) if R¹ and R⁴ are H and n is 3, then R⁵ is not methyl or ethyl,
or a cosmetically acceptable salt thereof.

2. The compound according to claim 1, which is a compound of formula (I-A)

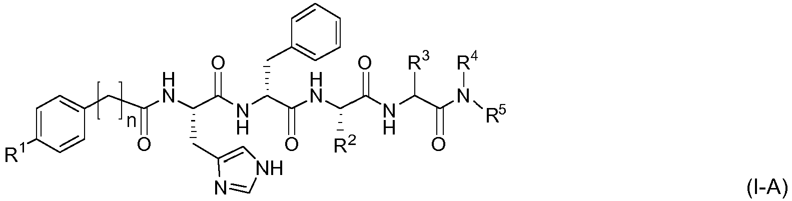

3. The compound according to claim 1 or 2, wherein R¹ is selected from H, C₁-C₂alkyl group or a C₁-C₂alkoxy group, preferably from H or methoxy.

4. The compound according to any one of claims 1 to 3, wherein R² is the amino acid side chain of arginine or diaminobutyric acid.

5. The compound according to any one of claims 1 to 4, wherein R³ is an aryl(m)ethyl group or an heteroaryl(m)ethyl group, preferably phenyl(m)ethyl, naphthyl(m)ethyl or (1H-indol-3-yl)(m)ethyl.

6. The compound according to any one of claims 1 to 5, wherein R⁴ and R⁵ are independently of each other selected from the group of H, benzyl and an unbranched C₁-C₁₀alkyl group, wherein the alkyl group may be substituted with up to two hydroxyl groups, preferably from the group of H, benzyl, propyl, butyl, octyl and 2,3-hydroxypropyl.

7. The compound according to any one of claims 1 to 6, wherein n is 2 or 3, preferably 3.

8. The compound of formula (I) according to claim 1, which is a compound of formula
| | |
|---|---|
| (I-a) | 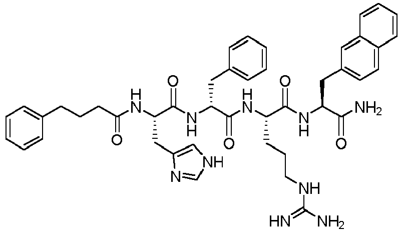 |
| (I-b) | 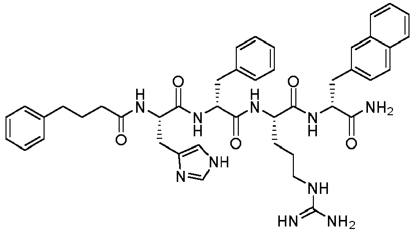 |
| (I-C) | 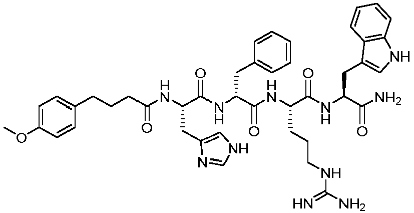 |
| (I-d) | 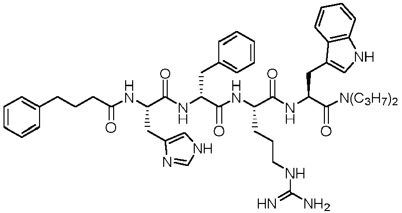 |
| (I-e) | 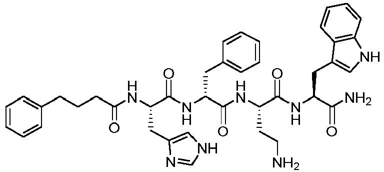 |
| (I-f) | 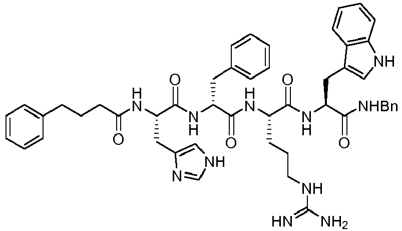 |
| (I-g) | 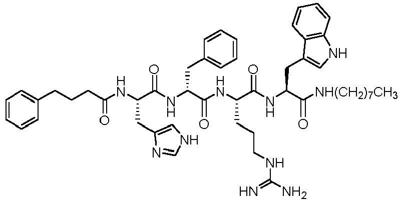 |
| (I-h) | 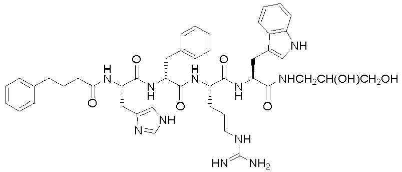 |
or a cosmetically acceptable salt thereof.

9. A cosmetic or pharmaceutical composition comprising at least one compound according to any one of claims 1 to 8 and a cosmetically acceptable carrier.

10. The cosmetic or pharmaceutical composition according to claim 9, wherein the total amount of the at least one compound of formula (I) is selected in the range of about 0.00001 to 0.5 wt.-%, more preferably in the range of 0.0001 to 0.25 wt.-%, most preferably in the range of 0.0001 to 0.1 wt.-% based on the total weight of the cosmetic composition.

11. The cosmetic or pharmaceutical composition according to claim 9 or 10, wherein the composition comprises at least one further ingredient selected from the group consisting of self-tanning agents, UV-filters, agents for the treatment of hyperpigmentation, agents for the prevention or reduction of inflammation, firming, moisturizing, soothing, and/ or energizing agents as well as agents to improve elasticity and skin barrier.

12. The cosmetic or pharmaceutical composition according to claim 9 to 11, wherein the composition further comprises at least on ingredient selected from the group consisting of polysilicones-15, phenylbenzimidazol sulfonic acid, 3-benzylidene camphor, octocrylene, ethylhexyl methoxycinnamate, ethyl hexylsalicylate, homosalate, zinc oxide, bis-ethylhexyloxyphenol methoxyphenyl triazine, methylene bis-benzotriazolyl tetramethylbutylphenol, titanium dioxide, butyl methoxydibenzoylmethane, erythrulose, potassium cetyl phosphate, tocopherol and/ or tocopherol acetate as well as mixtures thereof.

13. A composition according to any one of claims 9 to 12 for use in the treatment of the skin and/or scalp by contacting the skin and/or scalp with the composition.

14. The composition for use of claim 13 for maintaining a healthy skin homeostasis and/or skin microbiome balance.

15. A composition according to any one of claims 9 to 12 for maintaining a healthy skin homeostasis and/or skin microbiome balance.

16. A compounds according to any one of claims 1 to 8 for use as antimicrobial agent against Bacillus subtilis, and optionally Propionibacterium acnes and/or Staphylococcus aureus.

## Patentansprüche

1. Verbindung der Formel (I) 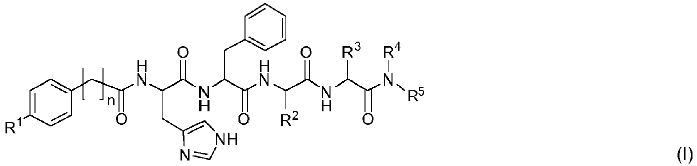 wobei
R¹ aus der Gruppe bestehend aus H, einer C₁-C₆-Alkylgruppe oder einer C₁-C₆-Alkoxygruppe ausgewählt ist,
R² für eine Aminosäure-Seitenkette einer basischen Aminosäure steht,
R³ für eine Aryl-C₁-C₆-alkylgruppe oder eine Heteroaryl-C₁-C₆-alkylgruppe steht,
R⁴ und R⁵ unabhängig voneinander für H, eine Aryl-C₁-C₆-alkylgruppe oder eine C₁-C₁₀-Alkylgruppe stehen, wobei die Alkylgruppe gegebenenfalls durch bis zu drei Hydroxygruppen substituiert ist, und
n für eine ganze Zahl steht, die aus 0 bis 3 ausgewählt ist,
mit der Maßgabe, dass
dann, wenn R² für die Aminosäure-Seitenkette von Arginin steht und R³ für (1H-Indol-3-yl)methyl steht,
(1) wenn R⁴ und R⁵ für H stehen und n für eine ganze Zahl von 0 bis 3 steht, R¹ nicht für H steht, oder
(2) wenn R⁴ und R⁵ für H stehen und n für 0 steht, R¹ nicht für Butoxy steht, oder
(3) wenn R¹ und R⁴ für H stehen und n für 3 steht, R⁵ nicht für Methyl oder Ethyl steht,
oder ein kosmetisch unbedenkliches Salz davon.

2. Verbindung nach Anspruch 1, bei der es sich um eine Verbindung der Formel (I-A) 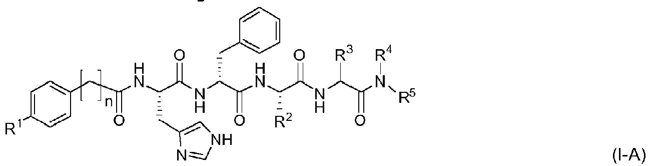 handelt.

3. Verbindung nach Anspruch 1 oder 2, wobei R¹ aus der Gruppe bestehend aus H, einer C₁-C₂-Alkylgruppe oder einer C₁-C₂-Alkoxygruppe, vorzugsweise aus H oder Methoxy, ausgewählt ist.

4. Verbindung nach einem der Ansprüche 1 bis 3, wobei R² für die Aminosäure-Seitenkette von Arginin oder Diaminobuttersäure steht.

5. Verbindung nach einem der Ansprüche 1 bis 4, wobei R³ für eine Aryl(m)ethylgruppe oder eine Heteroaryl(m)ethylgruppe, vorzugsweise Phenyl(m)ethyl, Naphthyl(m)ethyl oder (1H-Indol-3-yl)(m)ethyl, steht.

6. Verbindung nach einem der Ansprüche 1 bis 5, wobei R⁴ und R⁵ unabhängig voneinander aus der Gruppe H, Benzyl und einer unverzweigten C₁-C₁₀-Alkylgruppe, wobei die Alkylgruppe durch bis zu zwei Hydroxylgruppen substituiert sein kann, vorzugsweise aus der Gruppe H, Benzyl, Propyl, Butyl, Octyl und 2,3-Hydroxypropyl, ausgewählt ist.

7. Verbindung nach einem der Ansprüche 1 bis 6, wobei n für 2 oder 3, vorzugsweise 3, steht.

8. Verbindung der Formel (I) nach Anspruch 1, bei der es sich um eine Verbindung der Formel
| | |
|---|---|
| (I-a) | 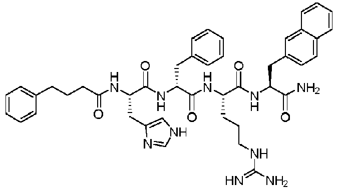 |
| (I-b) | 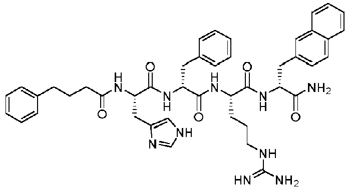 |
| (I-c) | 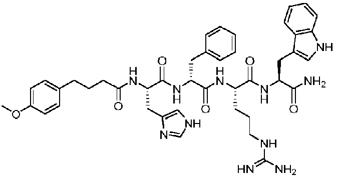 |
| (I-d) | 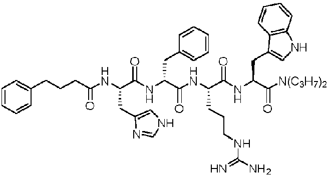 |
| (I-e) | 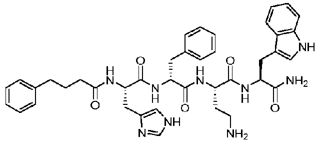 |
| (I-f) | 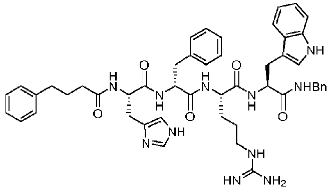 |
| (I-g) | 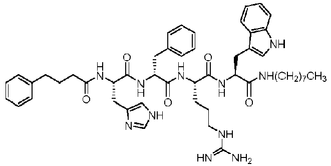 |
| (I-h) | 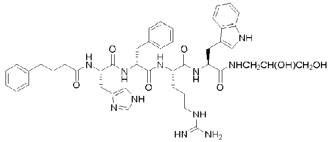 |
oder ein kosmetisch unbedenkliches Salz davon handelt.

9. Kosmetische oder pharmazeutische Zusammensetzung, umfassend mindestens eine Verbindung nach einem der Ansprüche 1 bis 8 und einen kosmetisch unbedenklichen Träger.

10. Kosmetische oder pharmazeutische Zusammensetzung nach Anspruch 9, wobei die Gesamtmenge der mindestens einen Verbindung der Formel (I) im Bereich von etwa 0,00001 bis 0,5 Gew.-%, weiter bevorzugt im Bereich von 0,0001 bis 0,25 Gew.-%, ganz besonders bevorzugt im Bereich von 0,0001 bis 0,1 Gew.-%, bezogen auf das Gesamtgewicht der kosmetischen Zusammensetzung, gewählt ist.

11. Kosmetische oder pharmazeutische Zusammensetzung nach Anspruch 9 oder 10, wobei die Zusammensetzung mindestens einen weiteren Bestandteil aus der Gruppe bestehend aus Selbstbräunungsmitteln, UV-Filtern, Mitteln zur Behandlung von Hyperpigmentierung, Mitteln zur Prävention oder Verringerung von Entzündung, festigenden, feuchtigkeitsspendenden, schmerzlindernden und/oder energiespendenden Mitteln sowie Mitteln zur Verbesserung von Elastizität und Hautbarriere umfasst.

12. Kosmetische oder pharmazeutische Zusammensetzung nach Anspruch 9 bis 11, wobei die Zusammensetzung ferner mindestens einen Bestandteil aus der Gruppe bestehend aus Polysilicones-15, Phenylbenzimidazolsulfonsäure, 3-Benzylidencampher, Octocrylen, Ethylhexylmethoxycinnamat, Ethylhexylsalicylat, Homosalat, Zinkoxid, Bis-ethylhexyloxyphenolmethoxyphenyltriazin, Methylenbis-benzotriazolyltetramethylbutylphenol, Titandioxid, Butylmethoxydibenzoylmethan, Erythrulose, Kaliumcetylphosphat, Tocopherol und/oder Tocopherolacetat sowie Mischungen davon umfasst.

13. Zusammensetzung nach einem der Ansprüche 9 bis 12 zur Verwendung bei der Behandlung der Haut oder Kopfhaut durch Inkontaktbringen der Haut und/oder Kopfhaut mit der Zusammensetzung.

14. Zusammensetzung zur Verwendung nach Anspruch 13 zur Aufrechterhaltung einer gesunden Hauthomöostase und/oder Hautmikrobiombalance.

15. Zusammensetzung nach einem der Ansprüche 9 bis 12 zur Aufrechterhaltung einer gesunden Hauthomöostase und/oder Hautmikrobiombalance.

16. Verbindungen nach einem der Ansprüche 1 bis 8 zur Verwendung als antimikrobielles Mittel gegen Bacillus subtilis und gegebenenfalls Propionibacterium acnes und/oder Staphylococcus aureus.

## Revendications

1. Composé de formule (I) 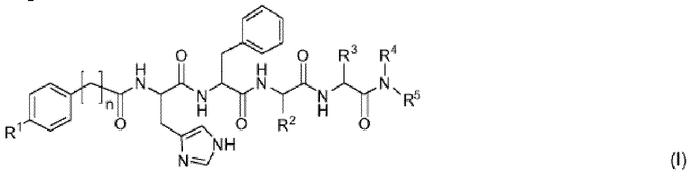
R¹ étant choisi parmi H, un groupe C₁₋₆alkyle et un groupe C₁₋₆alcoxy,
R² étant une chaîne latérale d'acide aminé d'un acide aminé basique,
R³ étant un groupe arylC₁₋₆alkyle ou un groupe hétéroarylC₁₋₆alkyle,
R⁴ et R⁵ étant, indépendamment l'un de l'autre H, un groupe arylC₁₋₆alkyle ou un groupe C₁₋₁₀alkyle, le groupe alkyle étant éventuellement substitué par jusqu'à trois groupes hydroxy, et
n étant un entier choisi parmi 0 à 3,
à la condition que
si R² est la chaîne latérale d'acide aminé de l'arginine et R³ est (1H-indol-3-yl)méthyle alors
(1) si R⁴ et R⁵ sont H et n est un entier de 0 à 3, alors R¹ n'est pas H, ou
(2) si R⁴ et R⁵ sont H et n est 0, alors R¹ n'est pas butoxy, ou
(3) si R¹ et R⁴ sont H et n est 3, alors R⁵ n'est pas méthyle ou éthyle,
ou sel acceptable sur le plan cosmétique correspondant.

2. Composé selon la revendication 1, qui est un composé de formule (I-A)

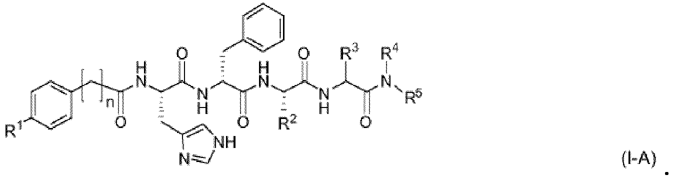

3. Composé selon la revendication 1 ou 2, R¹ étant choisi parmi H, un groupe C₁₋₂alkyle et un groupe C₁₋₂alcoxy, préférablement parmi H et méthoxy.

4. Composé selon l'une quelconque des revendications 1 à 3, R² étant la chaîne latérale d'acide aminé de l'arginine ou de l'acide diaminobutyrique.

5. Composé selon l'une quelconque des revendications 1 à 4, R³ étant un groupe aryl(m)éthyle, ou un groupe hétéroaryl(m)éthyle, préférablement phényl(m)éthyle, naphtyl(m)éthyle ou (1H-indol-3-yl)(m)éthyle.

6. Composé selon l'une quelconque des revendications 1 à 5, R⁴ et R⁵ étant indépendamment l'un de l'autre choisis dans le groupe composé de H, benzyle et un groupe C₁₋₁₀alkyle non ramifié, le groupe alkyle pouvant être substitué par jusqu'à deux groupes hydroxyle, préférablement dans le groupe composé de H, benzyle, propyle, butyle, octyle et 2,3-hydroxypropyle.

7. Composé selon l'une quelconque des revendications 1 à 6, n étant 2 ou 3, préférablement 3.

8. Composé de formule (I) selon la revendication 1, qui est un composé de formule
| | |
|---|---|
| (I-a) | 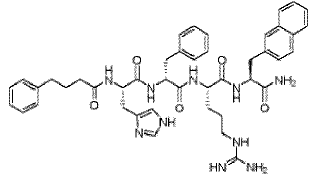 |
| (I-b) | 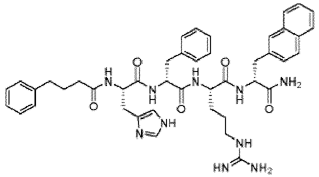 |
| (I-c) | 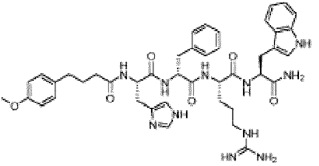 |
| (I-d) | 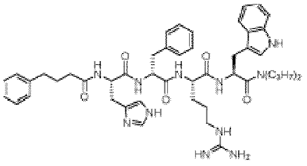 |
| (I-e) | 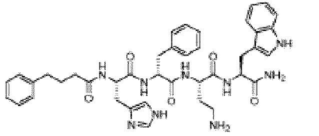 |
| (I-f) | 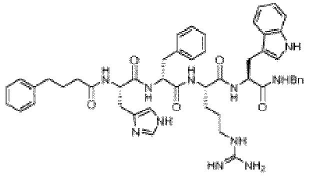 |
| (I-g) | 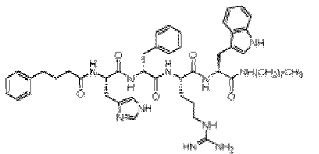 |
| (I-h) | 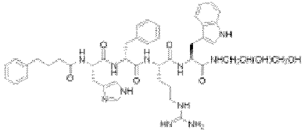 |
ou sel acceptable sur le plan cosmétique correspondant.

9. Composition cosmétique ou pharmaceutique comprenant au moins un composé selon l'une quelconque des revendications 1 à 8 et un support acceptable sur le plan cosmétique.

10. Composition cosmétique ou pharmaceutique selon la revendication 9, la quantité totale de l'au moins un composé de formule (I) étant choisie dans la plage d'environ 0,00001 à 0,5 % en poids, plus préférablement dans la plage de 0,0001 à 0,25 % en poids, le plus préférablement dans la plage de 0,0001 à 0,1 % en poids sur la base du poids total de la composition cosmétique.

11. Composition cosmétique ou pharmaceutique selon la revendication 9 ou 10, la composition comprenant au moins un ingrédient supplémentaire choisi dans le groupe constitué par des agents auto-bronzants, des filtres UV, des agents pour le traitement de l'hyperpigmentation, des agents pour la prévention ou la réduction d'une inflammation, des agents raffermissants, hydratants, apaisants et/ou énergisants ainsi que des agents pour améliorer l'élasticité et la barrière cutanée.

12. Composition cosmétique ou pharmaceutique selon les revendications 9 à 11, la composition comprenant en outre au moins un ingrédient choisi dans le groupe constitué par des polysilicones-15, l'acide phénylbenzimidazolsulfonique, le 3-benzylidènecamphre, l'octocrylène, le méthoxycinnamate d'éthylhexyle, l'hexylsalicylate d'éthyle, l'homosalate, l'oxyde de zinc, la biséthylhexyloxyphénol-méthoxyphényl-triazine, le méthylène-bis-benzotriazolyltétraméthylbutylphénol, le dioxyde de titane, le butyl-méthoxydibenzoylméthane, l'érythrulose, le cétylphosphate de potassium, le tocophérol et/ou l'acétate de tocophérol ainsi que des mélanges correspondants.

13. Composition selon l'une quelconque des revendications 9 à 12 pour une utilisation dans le traitement de la peau et/ou du cuir chevelu par mise en contact de la peau et/ou du cuir chevelu avec la composition.

14. Composition pour une utilisation selon la revendication 13 pour le maintien d'une homéostasie cutanée et/ou d'un équilibre microbiote cutané sain(e).

15. Composition selon l'une quelconque des revendications 9 à 12 pour le maintien d'une homéostasie cutanée et/ou d'un équilibre microbiote cutané sain(e).

16. Composés selon l'une quelconque des revendications 1 à 8 pour une utilisation en tant qu'agent antimicrobien contre Bacillus subtilis, et éventuellement Propionibacterium acnes et/ou Staphylococcus aureus.
